(19) Europäisches Patentamt
European Patent Office
Office européen des brevets

(11) Veröffentlichungsnummer : **0 127 827**
**B1**

(12) **EUROPÄISCHE PATENTSCHRIFT**

(45) Veröffentlichungstag der Patentschrift : **24.01.90**

(51) Int. Cl.$^5$ : **C 07 D239/30**

(21) Anmeldenummer : **84105727.6**

(22) Anmeldetag : **19.05.84**

(54) **Verfahren zur Herstellung von Chlorpyrimidinen.**

(30) Priorität : **01.06.83 DE 3319957**

(43) Veröffentlichungstag der Anmeldung : **12.12.84 Patentblatt 84/50**

(45) Bekanntmachung des Hinweises auf die Patenterteilung : **24.01.90 Patentblatt 90/04**

(84) Benannte Vertragsstaaten : **CH DE FR GB LI**

(56) Entgegenhaltungen :
**DE–A– 1 795 116**
**DE–A– 2 026 819**
**DE–A– 2 307 863**
**US–A– 3 629 261**

(73) Patentinhaber : **BAYER AG**
**D-5090 Leverkusen 1 Bayerwerk (DE)**

(72) Erfinder : **Beck, Gunther, Dr.**
**Am Mittelberg 19**
**D-5090 Leverkusen 1 (DE)**
Erfinder : **Heitzer, Helmut, Dr.**
**Höhenstrasse 84**
**D-5090 Leverkusen 3 (DE)**

**EP 0 127 827 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Die vorliegende Erfindung betrifft ein neues Verfahren zur Herstellung von Verbindungen der Formel

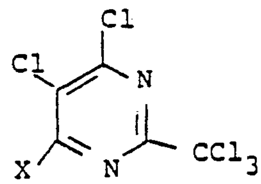

(I)

worin X für H oder Cl steht, dadurch gekennzeichnet, daß man N-(2-Cyanethyl)-acetamide der Formel

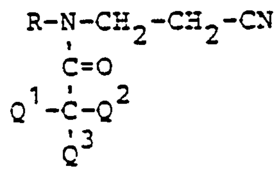

(II)

worin

R einen unter den Reaktionsbedingungen abspaltbaren Rest bedeutet, und

$Q^1$, $Q^2$, $Q^3$ gleich oder verschieden sein können und Wasserstoff und Chlor bedeuten,

in Mischung mit einem inerten Verdünnungsmittel zunächst bei Temperaturen von etwa 0 °C bis etwa 100 °C mit einem Säurechlorid behandelt und gleichzeitig oder anschließend bei Temperaturen von etwa 0 °C bis etwa 200 °C mit überschüssigem Chlor umsetzt und schließlich die beiden Komponenten (entsprechend Formel I, X = H und X = Cl) nach üblichen Methoden, insbesondere destillativ, auftrennt.

Verfahren zur Herstellung von Verbindungen der Formel (I) sind aus der Literatur bekannt. Siehe dazu US-A 3 629 261 sowie DE-A 2 026 819. Mit Hilfe des erfindungsgemäßen Verfahrens sind jedoch gemäß einem unerwarteten Reaktionsablauf und in guter Ausbeute die Verbindungen der Formel (I) leicht zugänglich.

Geeignete Reste R, die unter den Reaktionsbedingungen abspaltbar sind, sind insbesondere niederes Alkyl, vorzugsweise $C_1$-$C'_4$-Alkyl wie Methyl, Ethyl, Propyl, Butyl, weiterhin niederes, insbesondere $C_3$-$C_4$-Alkenyl wie Allyl, wobei diese Gruppen auch substituiert sein können, beispielsweise durch Chlor, Hydroxy, $C_1$-$C_4$-Alkoxy oder gegebenenfalls substituiertes Phenyl.

Geeignete Reste dieser Art sind beispielsweise Chlormethyl, Chlorethyl, Hydroxyethyl, Methoxyethyl, Benzyl, Phenylethyl, Chlorpropyl, Dichlorpropyl, Methoxypropyl.

Die Ausgangsverbindungen der Formel (II) sind nur zum Teil bekannt. Sie lassen sich jedoch leicht nach den Angaben der Literatur (z. B. Acta Polon. Pharm. 22, 219 (1964) und J. Chem. Soc., Perkin Trans. 2 1978, 1130) herstellen, indem man cyanethylierte Amine (III),

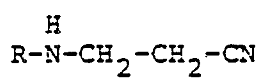

(III)

in denen R die obengenannte Bedeutung besitzt, mit einer aktivierten Form der Säuren (IV),

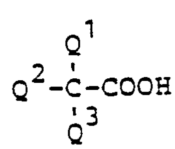

(IV)

in denen $Q^1$-$Q^3$ die obengenannten Bedeutungen besitzen, acyliert. Beispielsweise für aktivierte Formen der Säuren (IV) sind insbesondere die Anhydride und die Säurechloride von Essigsäure, Chloressigsäure, Dichloressigsäure und Trichloressigsäure.

Wählt man 3-(Methylamino)-propionitril als cyanethyliertes Amin (III) und Acetanhydrid als aktivierte Form einer der Säuren (IV), so ergibt sich folgendes Formel-schema :

(Siehe Formelschema Seite 3 f.)

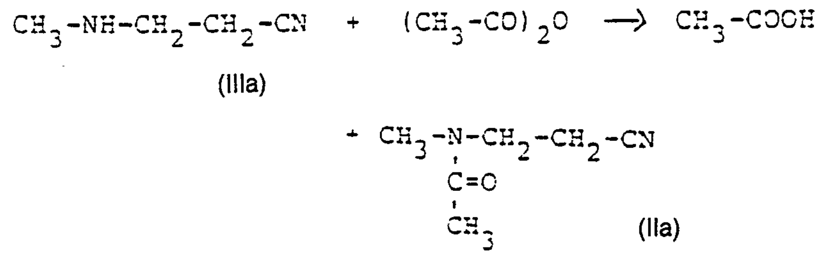

(IIIa)

(IIa)

Die cyanethylierten Amine (III) erhält man beispielsweise nach folgendem Formelschema

$$\underset{(V)}{R-NH_2} + CH_2=CH-CN \longrightarrow \underset{(III)}{R-NH-CH_2-CH_2-CN} ,$$

indem man primäre Amine (V), in denen R die obengenannte Bedeutung besitzt, an Acrylnitril anlagert

(vgl. z. B. J. Am. Chem. Soc. 66, 725 (1944)
J. Am. Chem. Soc. 68, 1217 (1946)
J. Am. Chem. Soc. 78, 2573 (1956)
J. Heterocyclic Chem. 1, 260 (1964)
J. Chem. Soc., Perkin Trans. 2 1978, 1130).

Für das erfindungsgemäße Verfahren geeignete N-(2-Cyanethyl)-acetamide der Formel (II) sind z. B. :

N-(2-Cyanethyl)-N-methyl-acetamid,
N-(2-Cyanethyl)-N-ethyl-acetamid,
N-(2-Cyanethyl)-N-allyl-acetamid,
N-(2-Cyanethyl)-N-benzyl-acetamid,
N-(2-Cyanethyl)-N-methyl-chloracetamid,
N-(2-Cyanethyl)-N-methyl-dichloracetamid,
N-(2-Cyanethyl)-N-methyl-trichlor-acetamid,
N-(2-Cyanethyl)-N-ethyl-chlor- bzw. -dichlor- bzw. trichlor-acetamid,
N-(2-Cyanethyl)-N-allyl-chlor- bzw. -dichlor- bzw. -trichlor-acetamid,
N-(2-Cyanethyl)-N-benzyl-chlor- bzw. -dichlor- bzw. -trichlor-acetamid,
N-(2-Cyanethyl)-N-propyl- bzw. -butyl-acetamid,
N-(2-Cyanethyl)-N-(2-chlorethyl)-acetamid,
N-(2-Cyanethyl)-N-(2-chlorethyl)-chlor- bzw. -dichlor- bzw. -trichlor-acetamid.

Verdünnungsmittel, die unter den Reaktionsbedingungen inert sind, sind alle gegen Chlor beständigen Lösungsmittel, z. B. chlorierte aliphatische und aromatische Kohlenwasserstoffe, wie Chloroform, Tetrachlorkohlenstoff, 1,1,2,2-Tetrachlorethan, 1,1,2,3,3-Pentachlorpropan, Hexachlorcyclopentadien, Oktachlorcyclopenten, 1,2,4-Trichlorbenzol, chlorierte Pyrimidine sowie Phosphoroxychlorid und Thionylchlorid. Im allgemeinen werden pro Volumteil (II) 0,5 bis 20, vorzugsweise 1 bis 10 Volumteile Verdünnungsmittel angewandt.

Geeignete Säurechloride sind vorzugsweise Chloride anorganischer Säuren wie Phosgen, Phosphorpentachlorid, Phosphortrichlorid, Phosphoroxychlorid und Thionylchlorid. Als organisches Säurechlorid sei beispielsweise Oxalylchlorid genannt.

Für eine vollständige Umsetzung der N-(2-Cyanethyl)-acetamide (II) ist die Anwendung von mindestens 1 Mol des Säurechlorids pro Mol (II) erforderlich. Im allgemeinen werden zwischen 1 und 2 Mol Säurechlorid pro Mol (II) eingesetzt. Phosphoroxychlorid und Thionylchlorid können dabei auch als Verdünnungsmittel verwendet werden. Darüber hinaus kann Phosphoroxychlorid auch ausschließlich als Verdünnungsmittel eingesetzt werden, wenn der bei 0 bis 30 °C frisch bereiteten Mischung aus $POCl_3$ und (II) ein reaktiveres Säurechlorid, z. B. Phosgen, Oxalylchlorid oder Phosphorpentachlorid, zugefügt wird bzw. wenn man dem $POCl_3$ zuerst einen Teil oder die Gesamtmenge des reaktiveren Säurechlorids zufügt und anschließend (II) portionsweise, gegebenenfalls gleichzeitig mit restlichem Säurechlorid, einbringt.

Für die an die Säurechlorid-Behandlung anschließende Umsetzung mit Chlor wird letzteres zweckmäßigerweise im Überschuß (erkennbar an der grünlichen Farbe des Chlorierungs-Abgases) angewendet, jedoch ist bei allen exotherm verlaufenden Chlorierungsphasen, insbesondere bei der Chlorierungs- Anfangsphase, überschüssiges Chlor nicht von Vorteil, da andernfalls die exotherme Reaktion zu heftig wird.

Die insgesamt mindestens einzusetzenden Mengen Chlor sind 6 Mol pro Mol (II), jedoch wird im allgemeinen ein größerer Überschuß angewandt, der bis das zehnfache der Mindestmenge betragen kann. Besonders bevorzugt wird jedoch so verfahren, daß beginnend bei Raumtemperatur — so lange bei

der jeweiligen Reaktionstemperatur, gegebenenfalls unter Kühlung, Chlor eingeleitet wird, bis z. B. durch die Grünfärbung des Abgases das Ende oder zumindest das Abklingen der Chloraufnahme angezeigt wird und dann erst die Reaktionstemperatur weiter erhöht wird.

Das Verfahren wird im einzelnen in der Weise durchgeführt, daß man zünachst ein N-(2-Cyanethyl)-acetamid der Formel (II), insbesondere das N-(2-Cyanethyl)-N-methylacetamid, mit einem der erwähnten Verdünnungsmittel, z. B. Phosphoroxychlorid, bei 0-30 °C mischt und anschließend mit mindestens 1 Mol eines vorzugsweise anorganischen Säurechlorids, vorzugsweise 1 bis 2 Mol, reagieren läßt. Die untere Grenze des Temperaturbereichs (etwa 0 bis 100 °C) ist von der Reaktivität des eingesetzten Säurechlorids abhängig. Phosgen, Phosphorpentachlorid und Oxalylchlorid beispielsweise reagieren bereits bei Raumtemperatur oder wenig darüber in exothermer Reaktion, so daß insbesondere dann eine Kühlung zweckmäßig ist, wenn man Phosphoroxychlorid ausschließlich als Verdünnungsmittel verwenden will und seine bei erhöhten Temperaturen einsetzende Funktion als Säurechlorid verhindern will. Bei Phosphoroxychlorid und Thionylchlorid in ihrer Eigenschaft als Säurechlorid liegt die untere Temperaturgrenze, bei der eine rasche Reaktion eintritt, bei etwa 50 °C ; man kann hier zweckmäßig bis zum jeweiligen Siedepunkt erhitzen.

Wie bereits angedeutet, kann man im Falle des als ausschließliches Verdünnungsmittel besonders bevorzugten Phosphoroxychlorids auch das Säurechlorid ganz oder teilweise vorlegen und (II) anteilweise, gegebenenfalls unter Kühlung vorzugsweise zwischen 0 und 50 °C zufügen.

Im weiteren Verlauf des Verfahrens kann man nun in verschiedener Weise vorgehen. Eine Möglichkeit ist z. B. die, erst nach völligem Abschluß der Reaktion von (II) mit dem Säurechlorid Chlor im Temperaturbereich von etwa 0 bis etwa 60 °C einzuleiten. Da die Anfangsreaktion des mit Säurechlorid behandelten (II) mit Chlor exotherm verläuft, ist zur Vermeidung von unerwünschten Nebenreaktionen in dieser Anfangs-Chlorierungsphase ein Temperaturbereich von 10 bis 40 °C bevorzugt, der sowohl durch Kühlung als auch durch nicht zu rasches Einleiten von Chlor zu halten ist. Nach dem Abklingen dieser ersten stärker exothermen Stufe wird Chlor deutlich langsamer aufgenommen ; in den Phasen langsamer Chloraufnahme kann man den Chlorstrom zweckmäßig so regeln, daß stets ein geringer Überschuß (grünlich gefärbtes Abgas) vorhanden ist.

Eine weitere Möglichkeit besteht darin, daß man die Säurechlorid-Behandlung und die erwähnte erste Chlorierungsphase praktisch gleichzeitig — gegebenenfalls nach Vorgabe eines Anteils des Säurechlorids — ablaufen läßt. Hierbei kann man wieder entweder das Säurechlorid vorlegen und (II) sowie Chlor gleichzeitig einspeisen oder (II) vorlegen und Säurechlorid und Chlor gleichzeitig oder etwas zeitversetzt zudosieren, oder auch (z. B. im Falle von Phosgen) Phosgen, (II) und Chlor praktisch gleichzeitig in vorgelegtes Verdünnungsmittel einbringen.

Als Produkte der Erfindungsgemäßen Chlorierungsreaktion erhält man im allgemeinen Gemische aus 4,5-Dichlor-2-(trichlormethyl)-pyrimidin und 4,5,6-Trichlor-2-(trichlormethyl)-pyrimidin in guter Gesamtausbeute.

Das Mengenverhältnis 4,5-Dichlor-2-(trichlormethyl)-pyrimidin/4,5,6-Trichlor-2-(trichlormethyl)-pyrimidin ist durch die Temperaturführung während der Chlorierung beeinflußbar. Verlängerung der Reaktionszeiten und Erhöhung der relativen Chlormenge in einem mittleren Temperaturbereich (etwa 50-70 °C) führt zu einer Erhöhung des Anteils an 4,5,6-Trichlor-2-(trichlormethyl)-pyrimidin, der auf über 90 Gew.-% gesteigert werden kann. Naturgemäß hängt der Zeitbedarf zur Erzeugung eines hohen Anteils an 4,5,6-Trichlor-2-(trichlormethyl)-pyrimidin bei gleichbleibend dimensionierten Chlor-Dosiervorrichtungen (z. B. Glaseinleitrohren im Labormaßstab) von der Ansatzgröße ab. Selbstverständlich wird der Zeitbedarf auch dann erhöht, falls zwischenzeitlich Niederschläge auftreten. Unter optimalen Bedingungen (ausschließlich Flüssigphase, hohe relative Chlormenge durch relativ geringe Ansatzgröße) sind mindestens 6 Stunden Chlorierungsdauer in dem mittleren Temperaturbereich von etwa 50 bis etwa 70 °C zur Erzeugung eines mindestens 90 %igen Gewichtsanteils an 4,5,6-Trichlor-2-(trichlormethyl)-pyrimidin erforderlich.

Wünscht man die Herstellung eines Pyrimidin-Gemisches mit einem relativ hohen Anteil an 4,5-Dichlor-2-(trichlormethyl)-pyrimidin, so muß die Temperaturführung während der Chlorierung so gestaltet werden, daß der oben erwähnte mittlere Temperaturbereich von etwa 50 bis etwa 70 °C möglichst rasch durchlaufen oder übersprungen wird. Hierzu kann man nach Beendigung der ersten exothermen Chlorierungsphase unterhalb 50 °C, insbesondere 20-40 °C, ohne weitere Chlorzufuhr bis auf Temperaturen oberhalb etwa 70 °C, insbesondere 80-130 °C, erhitzen und dann wieder chlorieren bis zur Beendigung der Chloraufnahme. Nach dieser Maßnahme sind Anteile von über 90 Gew.-% an 4,5-Dichlor-2-(trichlormethyl)-pyrimidin im Gemisch 4,5-Dichlor-2-(trichlormethyl)-pyrimidin/4,5,6-Trichlor-2-(trichlormethyl)-pyrimidin erhätlich. Es ist sogar möglich, nach beendeter Säurechlorid-Umsetzung von (II) direkt oberhalb etwa 70 °C, insbesondere bei 80-130 °C, bis zur beendeten Chloraufnahme (allerdings unter verminderter Gesamtausbeute der beiden Pyrimidine 4,5-Dichlor-2-(trichlormethyl)-pyrimidin und 4,5,6-Trichlor-2-(trichlormethyl)-pyrimidin) zu chlorieren.

Die erfindungsgemäß erhaltenen Gemische aus 4,5-Dichlor-2(trichlormethyl)-pyrimidin und 4,5,6-Trichlor-2-(trichlormethyl)-pyrimidin lassen sich im analytischen Maßstab leicht gaschromatographisch bestimmen und im präparativen Maßstab durch fraktionierte Destillation an einer geeigneten Kolonne auftrennen.

Wenn als Endprodukt hauptsächlich 4,5,6-Trichlor-2-(trichlormethyl)-pyrimidin erhalten werden soll,

kann man außer der oben geschilderten Möglichkeit auch noch den aus der US-Patentschrift 3 629 261 bekannten Weg wählen, indem man den Anteil an 4,5-Dichlor-2-(trichlormethyl)-pyrimidin entweder im Chlorierungsgemisch mit 4,5,6-Trichlor-2-(trichlormethyl)-pyrimidin oder nach vorheriger destillativer Abtrennung im Temperaturbereich um 200 °C unter UV-Bestrahlung zum 4,5,6-Trichlor-2-(trichlormethyl-)-pyrimidin nachchloriert.

4,5-Dichlor-2-trichlormethyl)-pyrimidin (farblose Nadeln aus Petrolether vom Schmelzpunkt 71 bis 72 °C) und 4,5,6-Trichlor-2-(trichlormethyl)-pyrimidin (farblose Kristalle aus Methanol vom Schmelzpunkt 63 bis 64 °C) sind in allen Eigenschaften mit Vergleichspräparaten (DE-A- 2 026 819, US-A 3 629 261) identisch.

4,5-Dichlor-2-(trichlormethyl)-pyrimidin ist ein Ausgangsstoff zur Herstellung von Herbiziden (DE-A 2 026 819) ; 4,5,6-Trichlor-2-(trichlormethyl)-pyrimidin ist ein Zwischenprodukt bei der Herstellung von Reaktivfarbstoffen, wobei die letztgenannte Verbindung mit einem chromophoren System unter Abspaltung von Chlorwasserstoffsäure nach folgendem Schema umgesetzt wird

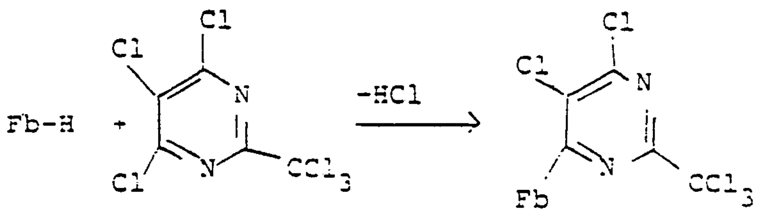


wobei Fb für den Rest eines chromophoren Systems steht.

Bei den in den folgenden Beispielen angegebenen Prozentwerten handelt es sich in allen Fällen um Gewichtsprozente.

Beispiel 1

In eine Mischung aus 1 000 ml 1,1,2,2-Tetrachlorethan und 500 g (3,97 Mol) N-(2-Cyanethyl)-N-methyl-acetamid wurden im Temperaturbereich von 20-30 °C im Verlauf von 3 Stunden 600 g (6,06 Mol) Phosgen eingeleitet. Anschließend wurde Chlor eingeleitet, wobei Chlorstrom und Kühlung so aufeinander abgestimmt wurden, daß sich die Reaktionstemperatur in dieser exotherm verlaufenden Anfangsphase zwischen etwa 25 und 35 °C hielt. Nach dem Abklingen dieser ersten Chlorierungsphase (etwa 7 Stunden) wurde unter Einleiten von leicht überschüssigem Chlor (erkennbar an der grünlichen Farbe des Abgases) im Verlauf von etwa 12 Stunden bis auf 112 °C aufgeheizt. Nach dem Abziehen des Lösungsmittels wurden im Wasserstrahlvakuum alle bis zu einer Badtemperatur von etwa 220 °C destillierbaren Anteile abdestilliert. Das farblose Destillat (950 g) enthielt nach der gaschromatographischen Analyse 31,4 % 4,5-Dichlor-2-(trichlormethyl)-pyrimidin und 65,0 % 4,5,6-Trichlor-2-(trichlormethyl-)-pyrimidin. Gesamt-Pyrimidin-Ausbeute 80 % der Theorie.

Beispiel 2

In eine Mischung aus 1 000 ml alkoholfreiem Chloroform und 500 g (3,97 Mol) N-(2-Cyanethyl)-N-methyl-acetamid wurden zwischen 30 und 50 °C in 3 Stunden 640 g (6,46 Mol) Phosgen eingeleitet. Anschließend wurde, beginnend bei 30 °C unter Eiskühlung, Chlor eingeleitet. Im Verlauf von 4 Stunden ab Chlorierungsbeginn wurde bis auf 60 °C gesteigert. Zwischendurch fiel bei etwa 30 °C ein Niederschlag aus, der bei etwa 58 °C wieder in Lösung ging. Nach weiterem sechsstündigem Einleiten von Chlor zwischen 60 und 65 °C wurde unter gleichzeitigem Abdestillieren des Chloroforms im Chlorstrom innerhalb 3,5 Stunden auf 176 °C gesteigert und dort noch weitere 2 Stunden chloriert. Das analog Beispiel 1 erhaltene Destillat (1 068 g) bestand nach der gaschromatographischen Analyse zu 53,6 % aus 4,5-Dichlor-2-(trichlormethyl)-pyrimidin und zu 36,1 % aus 4,5,6-Trichlor-2-(trichlormethyl)-pyrimidin. Gesamt-Pyrimidin-Ausbeute 86,4 % der Theorie.

Beispiel 3

In eine gekühlte Mischung aus 250 ml Phosphoroxychlorid und 50 g (0,397 Mol) N-(2-Cyanethyl)-N-methyl-acetamid wurden zwischen etwa 15 und 25 °C zunächst etwa 60 g (0,65 Mol) Phosgen eingeleitet. Anschließend wurde, beginnend bei 25 °C und unter Eiskühlung, Chlor eingeleitet. Unter allmählicher Temperatursteigerung wurden innerhalb 2 Stunden 55 °C erreicht und anschließend zwischen 55 und 66 °C 6 Stunden lang überschüssiges Chlor (erkennbar an der grünlichen Farbe des Abgases) eingeleitet. Anschließend wurde innerhalb 1,5 Stunden bis auf 103 °C gesteigert und dort noch weitere 2,5 Stunden chloriert. Der Chlorierungsansatz war stets homogen. Das analog Beispiel 1 erhaltene Destillat (90 g) enthielt 5,3% 4,5-Dichlor-2-(trichlormethyl)-pyrimidin und 94,0 % 4,5,6-Trichlor-2-(trichlormethyl)-pyrimidin. Gesamt-Pyrimidin-Ausbeute : 75,3 % der Theorie.

Beispiel 4

In eine Mischung aus 770 g (6,03 Mol) 98,8 %iges N-(2-Cyanethyl)-N-methyl-acetamid und 1 750 ml 1,1,2,2-Tetrachlorethan wurden zwischen 20 und 30 °C 1 190 g (ca. 12 Mol) Phosgen eingeleitet. Anschließend wurde, anfangs unter Eiskühlung, beginnend bei 22 °C Chlor eingeleitet und nach Abkühlen der exothermen Reaktionsphase langsam im Chlorstrom aufgeheizt. Nach achtstündiger Chlorierungsdauer war eine Temperatur von 64 °C erreicht; bei dieser Temperatur wurde dann weitere 14 Stunden lang leicht überschüssiges Chlor eingeleitet. Destillation analog Beispiel 1 erbrachten 1 043 g Destillat, das nach der gaschromatographischen Analyse zu 33,1 % aus 4,5-Dichlor-2-(trichlormethyl)-pyrimidin und zu 51,8 % aus 4,5,6-Trichlor-2-(trichlormethyl)-pyrimidin bestand. Gesamt-Pyrimidin-Ausbeute 57 % der Theorie.

Beispiel 5

250 ml Phosphoroxychlorid wurden mit 63,0 g (0,5 Mol) N-(2-Cyanethyl)-N-methyl-acetamid versetzt. Die Mischung wurde zunächst eine Stunde auf 90-95 °C erwärmt, anschließend auf 25 °C abgekühlt und Chlor eingeleitet. Nach dem Abklingen der exothermen Reaktion und Grünlichfärbung des Abgases (Chlorverbrauch ca. 270 g) wurde unter allmählicher Temperatursteigerung innerhalb von etwa 6 Stunden bis auf Rückflußtemperatur (ca. 107 °C) gesteigert. Gesamt-Chlorverbrauch ca. 1 190 g. Destillation analog Beispiel 1 erbrachten 111,2 g farbloses Destillat, das nach der gaschromatographischen Analyse zu 57,9 % aus 4,5-Dichlor-2-(trichlormethyl)-pyrimidin und zu 31,2 % aus 4,5,6-Trichlor-2-(trichlormethyl)-pyrimidin bestand. Gesamt-Pyrimidin-Ausbeute 71,4 % der Theorie.

Beispiel 6

Eine Suspension von 115 g (ca. 0,55 Mol) Phosphorpentachlorid in 200 ml Phosphoroxychlorid wurde unter Rühren ohne Kühlung in solchen Anteilen mit 63,0 g (0,5 Mol) N-(2-Cyanethyl)-N-methyl-acetamid versetzt, daß eine Temperatur von etwa 50 °C erreicht wurde. Anschließend wurde auf etwa 20 °C gekühlt und Chlor eingeleitet. Chlorstrom und Kühlung wurden so aufeinander abgestimmt, daß sich die Reaktionstemperatur zwischen etwa 20 und etwa 40 °C hielt. Nach dem Abklingen der ersten exothermen Chlorierungsphase wurde unter Einleiten von meist überschüssigem Chlor im Verlauf von etwa 6 Stunden bis auf Rückflußtemperatur (ca. 106 °C) aufgeheizt. Zwischendurch fiel bei etwa 24 °C ein Niederschlag aus, der bei 58 °C wieder in Lösung ging. Gesamt-Chlorverbrauch ca. 900 g. Destillation analog Beispiel 1 lieferte 156,8 g Destillat, das nach der geaschromatographischen Analyse 36,5 % 4,5-Dichlor-2-(trichlormethyl)-pyrimidin und 22,0 % 4,5,6-Trichlor-2-(trichlormethyl)-pyrimidin enthielt. Gesamt-Pyrimidin-Ausbeute 65,9 % der Theorie.

Beispiel 7

Eine Suspension von 230 g (ca. 1,1 Mol) Phosphorpentachlorid in 400 ml Phosphoroxychlorid wurde mit 126 g (1 Mol) N-(2-Cyanethyl)-N-methyl-acetamid versetzt und die Mischung anschließend auf 92 °C bis zum Ende der Gasentwicklung erwärmt. Anschließend wurden zwischen 90 und 95 °C im Verlauf von 4 Stunden 470 g Chlor eingeleitet. Das analog Beispiel 1 erhaltene Destillat enthielt nach der gaschromatographischen Analyse 62,3 g 4,5-Dichlor-2-(trichlormethyl)-pyrimidin und 3,6 g 4,5,6-Trichlor-2-(trichlormethyl)-pyrimidin. Gesamt-Pyrimidin-Ausbeute 24,6 % der Theorie. Anteil an 4,5-Dichlor-2-(trichlormethyl)-pyrimidin im Gemisch dieser beiden Pyrimidine ca. 94,6 Gewichtsprozent.

Beispiel 8

Eine Suspension von 115 g (ca. 0,55 Mol) Phosphorpentachlorid in 250 ml Phosphoroxychlorid wurde mit 63,0 g (0,5 Mol) N-(2-Cyanethyl)-N-methyl-acetamid versetzt, wobei das Reaktionsgemisch 15 Minuten auf 50 °C erwärmt wurde. Unter Kühlung wurde nun zwischen 20 und 30 °C ein Chlorstrom bis zum Ende der exothermen Reaktion und bis zur Grünfärbung des Abgases eingeleitet. (ca. 30 Minuten; ca. 120 g Chlorverbrauch). Anschließend wurde ohne weiteres Einleiten von Chlor auf 90-95 °C Innentemperatur erhitzt und dort so lange Chlor eingeleitet, bis das Abgas erneut eine Grünfärbung annahm (ca. 100 Minuten unter Verbrauch von weiteren 180 g Chlor).

Das analog Beispiel 1 erhaltene Destillat enthielt nach der gaschromatographischen Analyse 49,9 g 4,5-Dichlor-2-(trichlormethyl)-pyrimidin und 3,7 g 4,5,6-Trichlor-2-(trichlormethyl)-pyrimidin. Gesamt-Pyrimidin-Ausbeute 40,0 % der Theorie. Anteil an 4,5-Dichlor-2-(trichlormethyl)-pyrimidin im Gemisch dieser beiden Pyrimidine ca. 93 Gew.-%.

**Patentansprüche**

1. Verfahren zur Herstellung von Verbindungen der Formel

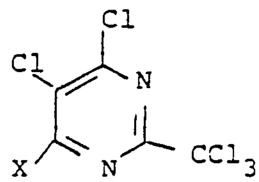

(I)

worin X für H oder Cl steht,
dadurch gekennzeichnet, daß man N-(2-Cyanethyl)-acetamide der Formel

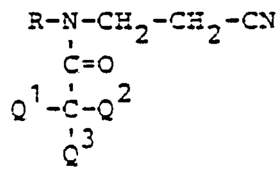

(II)

worin
R einen unter den Reaktionsbedingungen abspaltbaren Rest bedeutet,
und
$Q^1$, $Q^2$, $Q^3$ gleich oder verschieden sein können und Wasserstoff und Chlor bedeuten, in Mischung mit einem inerten Verdünnungsmittel zunächst bei Temperaturen von etwa 0 °C bis etwa 100 °C mit mindestens 1 Mol eines Säurechlorids behandelt und gleichzeitig oder anschließend bei Temperaturen von etwa 0 °C bis etwa 200 °C mit überschüssigem Chlor umsetzt und schließlich die beiden Komponenten (entsprechend Formel I, X = H und X = Cl) nach üblichen Methoden auftrennt.

2. Verfahren nach Anspruch 1, dadurch gekennzeichnet, daß man als Ausgangsverbindung N-(2-Cyanethyl)-N-($C_1$-$C_4$-alkyl)-acetamide einsetzt.

3. Verfahren nach Anspruch 1, dadurch gekennzeichnet, daß man als Ausgangsverbindung N-(2-Cyanethyl)-N-methylacetamid einsetzt.

4. Verfahren nach Anspruch 1 bis 3, dadurch gekennzeichnet, daß man als Säurechlorid Phosgen einsetzt.

5. Verfahren nach Anspruch 1-4, dadurch gekennzeichnet, daß man die Umsetzung mit dem Säurechlorid zwischen etwa 0 und etwa 100 °C durchführt.

6. Verfahren nach Anspruch 1-5, dadurch gekennzeichnet, daß man nach oder während der Umsetzung mit dem Säurechlorid bei etwa 0-60 °C, insbesondere bei 10-40 °C mit mindestens 6 Mol Chlor behandelt.

7. Verfahren nach Anspruch 6, dadurch gekennzeichnet, daß man mit mindestens 6 Mol Chlor pro Mol N-(2-Cyanethyl)-acetamid umsetzt.

**Claims**

1. Process for the preparation of compounds of the formula

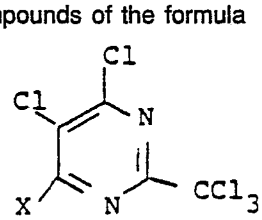

in which X represents H or Cl,
characterized in that N-(2-cyanoethyl)acetamides of the formula

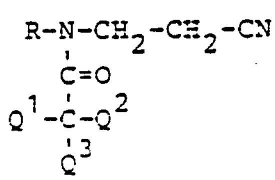

in which
R denotes a radical which is detachable under the reaction conditions,
and
$Q^1$, $Q^2$, $Q^3$ may be identical or different and denote hydrogen and chlorine, mixed with an inert

diluent are initially treated at temperatures from about 0 °C to about 100 °C with at least 1 mol of an acid chloride and are simultaneously or subsequently reacted at temperatures from about 0 °C to about 200 °C with an excess of chlorine and finally the two components (corresponding to formula I, X = H and X = Cl) are separated by customary methods.

2. Process according to Claim 1, characterized in that N-(2-cyanoethyl)-N-($C_1$-$C_4$-alkyl)acetamide is used as the starting compound.

3. Process according to Claim 1, characterized in that N-(2-cyanoethyl)-N-methylacetamide is used as starting compound.

4. Process according to Claim 1 to 3, characterized in that phosgene is used as acid chloride.

5. Process according to Claim 1-4, characterized in that the reaction with the acid chloride is carried out between about 0 and about 100 °C.

6. Process according to Claim 1-5, characterized in that after or during the reaction with the acid chloride, the mixture is treated with at least 6 mol of chlorine at about 0-60 °C, particularly at 10-40 °C.

7. Process according to Claim 6, characterized in that at least 6 mol of chlorine are reacted per mol of N-(2-cyanoethyl)acetamide.

**Revendications**

1. Procédé de production de composés de formule

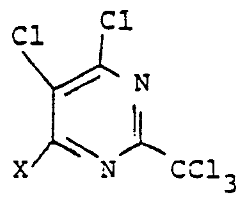

(I)

dans laquelle X représente H ou Cl,
caractérisé en ce qu'on traite tout d'abord avec au moins une mole d'un chlorure d'acide des N-(2-cyanéthyl)-acétamides de formule

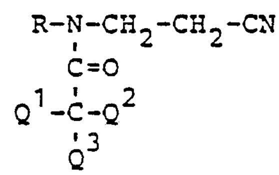

(II)

dans laquelle
R désigne un reste éliminable dans les conditions réactionnelles,
et
$Q^1$, $Q^2$, $Q^3$ peuvent être identiques ou différents et représentent de l'hydrogène et du chlore, en mélange avec un diluant inerte, tout d'abord à des températures d'environ 0 °C à environ 100 °C et on les fait réagir en même temps ou ensuite à des températures d'environ 0 °C à environ 200 °C avec du chlore en excès et, finalement, on sépare les deux composants (correspondant à la formule I, X = H et X = Cl) par des opérations classiques.

2. Procédé suivant la revendication 1, caractérisé en ce qu'on utilise comme composé de départ des N-(2-cyanéthyl)-N-(alkyle en $C_1$ à $C_4$)-acétamides.

3. Procédé suivant la revendication 1, caractérisé en ce qu'on utilise le N-(2-cyanéthyl)-N-méthylacétamide comme composé de départ.

4. Procédé suivant les revendications 1 à 3, caractérisé en ce qu'on utilise le phosgène comme chlorure d'acide.

5. Procédé suivant les revendications 1 à 4, caractérisé en ce qu'on conduit la réaction avec le chlorure d'acide entre environ 0 et environ 100 °C.

6. Procédé suivant les revendications 1 à 5, caractérisé en ce qu'on effectue le traitement avec au moins 6 moles de chlore après ou pendant la réaction avec le chlorure d'acide à une température d'environ 0 à 60 °C, notamment de 10 à 40 °C.

7. Procédé suivant la revendication 6, caractérisé en ce qu'on conduit la réaction avec au moins 6 moles de chlore par mole de N-(2-cyanéthyl)-acétamide.